# EUROPEAN PATENT APPLICATION

(11) **EP 4 245 353 A1**
(43) Date of publication of application: **20.09.2023**
(21) Application number: 23161103.9
(22) Date of filing: 10.03.2023
(51) Int. Cl.: A61M 39/10, A61M 39/18

(54) **REVERSIBLE STERILE CONNECTION SYSTEM**

(30) Priority: 14.03.2022 US 202263319613 P
(71) Applicant: Fenwal, Inc., Lake Zurich, IL 60047 (US)
(72) Inventor: MADSEN, James, Lake Zurich, 60047 (US); WEGENER, Christopher J., Lake Zurich, 60047 (US)
(74) Representative: Maikowski & Ninnemann Patentanwälte Partnerschaft mbB

(57) **Abstract**

Systems, devices and methods for establishing a sterile fluid flow path between two or more fluid processing sets are disclosed. The systems, devices and methods include two or more connectors to which tubes of two or more fluid processing sets are attached. The connectors are brought together in an air-tight, non-permanent engagement with one another and treated with sterilizing light.

## Description

### Field of the Disclosure

The present disclosure is directed to systems, devices, and methods for establishing a sterile fluid flow path between two or more fluid processing sets. More particularly, the present disclosure is directed to systems, devices, and methods for establishing a reversible, sterile connection between two or more fluid processing sets wherein the connection does not involve forming a physical and permanent sealing or welding of one set to another set.

### Background

In the medical field, the transfer of medical and/or biological fluids from a source container to one or more other containers must typically be carried out in a sterile manner, i.e., without exposing the fluids or the open ends of the tubing that carry the fluid to the outside environment. Accordingly, creating a sterile connection and establishing a sterile flow path between the source fluid and the receiving containers or receptacles is needed.

Currently, one way for making a sterile fluid flow connection is to use needleless connectors. Fluid flow connection using needleless connectors is an aseptic process where the exterior surface of the valve, diaphragm or septum of the female connector is manually swabbed with antiseptic prior to insertion of the male connector (which compresses and opens the valve). Manual swabbing requires care, increased vigilance and discipline on the part of the healthcare professional attending to a patient or carrying out the process in question. Failure to follow rigorous procedures can potentially result in microorganisms or other contaminants being inadvertently introduced into the fluid path. Even for specialized care in controlled environments, training and existing connector design may not completely eliminate the possibility of human error, which can result in contamination when such manual process is used.

Other ways for connecting medical fluid flow tubing include irradiation of connectors with ultraviolet light to provide an antibacterial effect on, or sterilization of, the irradiated connector surfaces. Examples of such systems and devices are presented in U.S. Patent Nos. to 4,500,788 to Kulin et al.; 4,503,333 to Kulin et al. and 4,883,496 to Bellotti et al. The systems and devices of these references, however, are intended for use in specialized settings with connectors of special design.

Another example of a device that creates a sterile connection and a sterile fluid pathway is the TSCD-II sterile tubing welder, available from Terumo Medical Corporation. This device uses a heated cutting element to sever and melt the ends of facing tubing ends which are joined together after the cutting element is removed. The heated wafer also sterilizes the connection. Aspects of this device are disclosed in U.S. Patent Application Publication No. 2020/0047423, which is incorporated herein by reference. Other sterile connection systems are described in U.S. Patent Application Publication No. US 2014/0077488; U.S. Patent No. 4,157,723; and U.S. Patent No. 5,009,446.

One disadvantage of a welding device and the method of making a sterile connection using such device is that the welded connection cannot be easily undone as the tubes are physically bonded to each other. Thus, where fluid from a single source container must be dispensed to multiple (smaller volume) containers or receptacles, (such as in the collection of multiple samples from a large volume of a collected biological fluid) each transfer from the source container would require severing the first connection between the source container and the receiving container, followed by the (re)establishing a new sterile connection between the source and the next receiving container. This sequence of creating a sterile connection, transferring fluid, severing the connection, and re-establishing a sterile connection with the next receiving container is time-consuming and inefficient.

In addition, the repeated severing of a tubing each time a subsequent sterile connection is required results in a further shortened tubing length in one of the fluid processing sets which may eventually make manipulating the tubing and the processing set more difficult and challenging.

Thus, it would be desirable to provide a device which can easily and repeatedly establish a sterile fluid pathway without the successive cutting, heating, and physical joining of plastic tubes. In addition, it would be desirable to provide a system and method that does not require manual user disinfection by swabs or the use of heater wafers.

The systems, devices and methods disclosed herein avoid the shortcomings of current sterile connection systems and address these and other needs.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a system for establishing a sterile fluid connection between a first fluid processing set and a second fluid processing set is provided. The system includes a first connector configured for attachment with a first fluid processing set and a second connector configured for attachment with a second processing set. The first connector includes a housing having a receiver at a distal end of the first connector. The receiver includes an open end and defines a chamber configured to receive at least a portion of the second connector in air-tight engagement. The system includes a light source in proximity to said first and second connectors.

In another aspect, a connector for establishing a reversible, sterile fluid connection between a first tube and a second tube is provided. The connector includes a body having a proximal end portion and a distal end portion. The connector includes a plunger moveable within said body and a sealing member associated and moveable with the plunger from a first sealing position to a second open flow position. A tube attachment member is located at the proximal end portion of the body. The connector includes an openable flow path between the attachment member and the plunger.

In a still further aspect, a method of establishing a sterile fluid connection between two or more fluid processing sets is provided. The method includes attaching a tube of a first fluid processing set to a proximal end of a first connector, the first connector including a plunger with a distal aperture and a sealing member. The sealing member is associated and moveable with the plunger from a first sealing position to a second open flow position. The method includes attaching a tube of a second processing set to a proximal end of a second connector, the second connector including a plunger with a distal eyelet and a sealing member. The sealing member is associated and moveable with the plunger from a first sealing position to a second open flow position. In a further step, the method includes aligning a distal end of the first connector plunger with a distal end of the second connector plunger in a face-to-face relationship and contacting said distal ends of the first and second connector plungers. In accordance with the method, each of the sealing members of the first and second connectors are moved from a first sealing position to an open flow position. The connectors are exposed to light from a light source for a selected period of time, the light being sufficient to sterilize the interiors of the first and second connectors. The method includes flowing fluid from a first processing set through said first and second connectors to said second processing set.

These and other aspects of the present subject matter are set forth in the following detailed description of the accompanying drawings.

### Brief Description of the Drawings

Figure 1 is a schematic view of the system and devices for establishing a sterile fluid flow path in accordance with the present disclosure;
Figure 2 is a perspective view of a first connector of the sterile connection system disclosed herein;
Figure 3 is an exploded view of the first connector of Fig, 2;
Figure 4 is a side view of the first connector of Fig. 2 with the plunger and sealing member in a first sealed position;
Figure 5 is a side view of the first connector of Fig. 2 with the plunger and sealing member in a second open flow position;
Figure 6 is a sectional view of the first connector of Fig. 2 showing the flow path through the first connector when said connector is in the open flow position;
Figure 7 is a perspective view of a second connector of the sterile connection system disclosed herein;
Figure 8 is an exploded view of the second connector of Fig. 7;
Figure 9 is a side view of the second connector of Fig. 7 with the plunger and sealing member in a first sealed position;
Fig. 10 is a sectional view of the second connector of Fig. 7 showing the flow path through the second connector when said connector is in the open flow position;
Fig. 11 is a side view of the sterile connection system during exposure of the connectors to light;
Fig. 12 is a side view of the sterile connection system wherein the first and second connectors are engaged and in the open flow position;
Fig. 13 is a sectional view of the sterile connection system of Fig. 12 showing the flow of fluid therethrough;
Fig. 14 is a side view of the sterile connection system wherein the first and second connectors are dis-engaged and the plungers and sealing members returned to their first sealed positions; and
Fig. 15 is side view of the sterile connection system wherein the first and second connectors are dis-engaged and separated from each other and ready for a subsequent sterilization cycle.

### Detailed Description of the Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

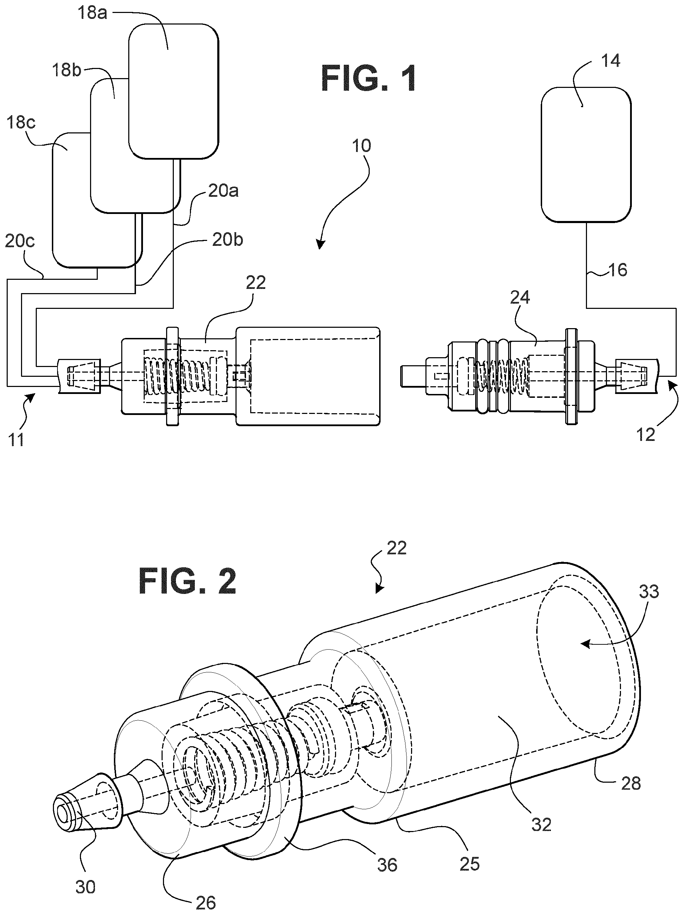

Fig. 1 schematically shows the components of a system 10 for establishing a sterile fluid connection between a first fluid processing set 11 and a second fluid processing set 12. As used herein, the term "fluid processing set" refers to any kit, fluid circuit, container, receptacle including its associated tubing for holding, receiving, and flowing a medical or biological fluid. In one example, a fluid processing set may include a container that holds a source of liquid that is to be transferred or dispensed from the source container. "Fluid processing set" may also refer to a tubing and container kit configured to receive a source fluid in one or more fluid receptacles. Other configurations of fluid processing sets may also be possible.

As shown in Fig. 1, fluid processing set 11 may include one or more receptacles 18 ((a)-(c)) for receiving source fluid from (source) container 14. Source container 14 includes tube 16 defining a flow path for the source liquid. Source container 14 may hold a medical or biological fluid such as blood, a blood component, a cell suspension, additive solution, anticoagulant, or any other fluid/liquid used in the processing of biological fluids that require sterile handling. Source liquid in container 14 may be dispensed to one or more containers 18 (a)-(c) of one or more fluid processing sets 11. Naturally, the transfer of the fluid must be done in a sterile manner.

As shown in Fig. 1, sterile transfer of fluid from fluid processing set 12 to fluid processing set 11 may be accomplished using the system described herein. System 10 allows for temporary association of the tubing 16 of processing set 12 and processing set 11 without physically sealing together the open ends of the tubings 16 and 20 (a, b, or c). To that end, system 10 includes connectors 22 and 24 that facilitate the establishment of a flow path between sets 11 and 12 and allow for the sterilization of the flow path by sterilizing medium such as light. While other forms of sterilization such as ethylene oxide gas, gamma radiation or autoclaving may be employed, sterilization with light (e.g., UV-C) may be preferred. Connectors 22 and 24 bring together the ends of tubings 16 and 20, or more specifically, the ends of connectors 22 and 24 without permanently joining the ends. Once sterilization of the connection site has been achieved, fluid transfer from container 14 to receptacles 18 may occur in a sterile manner. After fluid transfer is completed, connectors are withdrawn from each other and may be detached from their respective processing sets. Connectors 22 and 24 are reusable and once detached are ready for re-use (including re-sterilization) in a subsequent fluid transfer. For example, if fluid from source container 14 is to be sequentially dispensed to multiple receptacles 18 (a)-(c), once fluid has been transferred to a first receptacle 18(a), the connectors may be detached from another and a new connection between source container 14 and a receptacle 18(b) with a connector pre-attached to the tubing of receptacle 18(b) may be made. The process of bringing together connectors 22 and 24 (which is described in greater detail below) is repeated, as is the exposure of the temporary flow path to a sterilizing environment and the transfer of fluid from container 14 to container 18(b), 18(c), 18(d) etc.

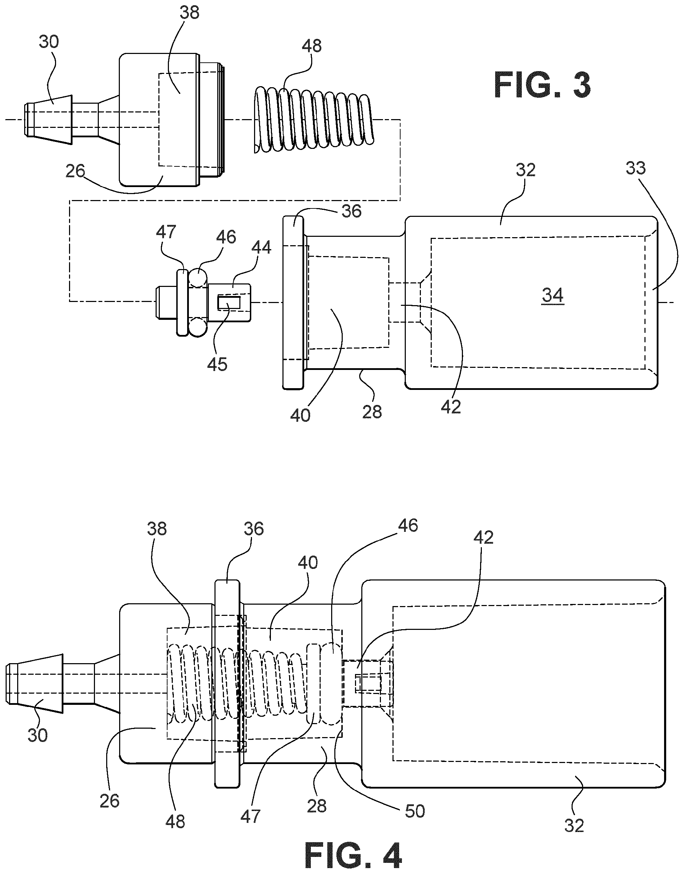

Turning now to Fig. 2, there is shown a connector 22 to which a tubing end of a processing set may be attached and used in accordance with the system and method described herein. As shown in Fig. 2 and in the exploded view of Fig. 3, connector 22 includes a generally cylindrically shaped housing 25 of varying outer diameter. Housing 25 includes a proximal housing portion 26 and a distal end portion 28. Proximal and distal housing portions may be provided as separate parts that are connected such as by solvent bonding, laser welding or threaded engagement at the time of assembly.

The terms "proximal" and "distal" are used to describe ends or portions of connector 22 (and connector 24 described below). As used herein, the term "proximal" refers to the end of a connector that is closer to the tubing of the processing set to which it is attached. The term "distal" refers to that portion or end of the connector that is further away from the tubing end and/or processing set to which the connector is attached. The "distal" end of a connector is that end that engages the other connector. Thus, during establishment of a sterile pathway between two processing sets, the distal ends of connectors 22 and 24 are brought together in a face-to-face manner, as shown in Fig. 1.

As further seen in Figs. 2 and 3, proximal portion of connector 22 includes an attachment member 30 that proximally extends from the proximal housing portion 26. Attachment member 30 defines an interior flow path that communicates with the interior of housing 25. The outer surface of attachment member 30 may be contoured or otherwise sized and shaped to receive the lumen of tubing associated with a processing set and to secure the tubing end to connector 22. Thus, as shown in Fig 2, attachment member 30 may include a barb for press fitting tube 20 (a, b or c) onto the attachment member 30. In lieu of a barb, attachment member 30 may include ribs, fins, luer lock or threads for receiving and securing tubing 20.

Distal housing portion 28 of connector 22 includes receiver 32 at its distal end. Receiver 32 may be a hollow cylinder with an open distal end 33. Receiver 32 defines a chamber 34 (Fig. 3) sized and shaped for receiving a distal end of connector 24. Connector 22 further includes collar 36 proximally spaced from receiver 32 at the proximal opening of distal housing portion 28.

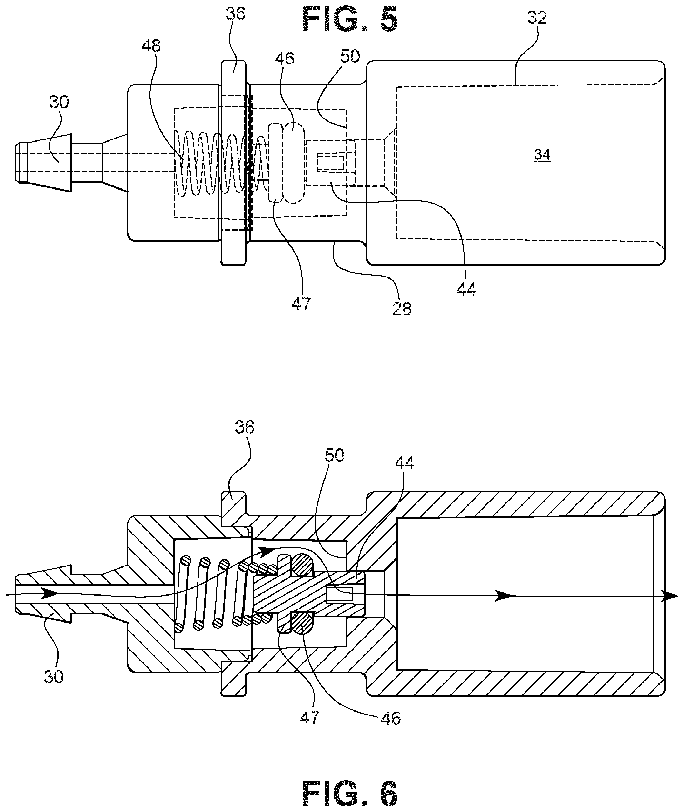

Figs. 3 and 4 show the internal components of connector 22. Both the proximal and distal housing portions 26 and 28 define hollow interiors 38 and 40 that accommodate the components described below. In addition, connector 22 further includes a passageway 42 that allows for axial movement of at least a portion of plunger 44 within passageway 42. Plunger 44 includes an aperture 45 at its distal tip end and a flange or collar 47 proximally spaced from said aperture. Flange 47 seats a resilient sealing member 46 such as an internal O-ring. Plunger 44 is attached to spring 48. As shown in Fig. 4, when spring 48 is in it expanded (uncompressed) condition sealing member 46 presses against wall 50 and seals passageway 42 thereby preventing liquid flow through connector 22. When spring 48 is compressed (see Fig. 5), sealing member 46 is retracted such that aperture 45 is open to flow and allows flow through connector 22. This is best seen in Fig. 6 and will be evident from the discussion that follows pertaining to the method of establishing a reversible sterile connection.

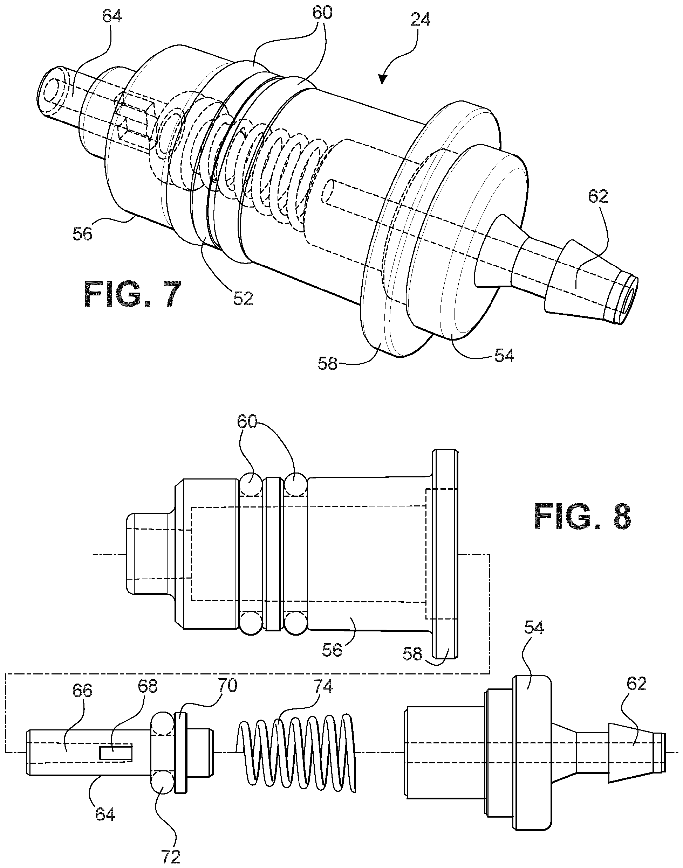

Fig. 7 shows the other (second) connector 24 of the connector pair for establishing a reversible sterile connection between two processing sets. As with connector 22, connector 24 includes a generally cylindrical housing 52 made of two portions, a proximal housing portion 54 and a distal housing portion 56 that may be attached to each other at the time of assembly by (solvent) bonding, laser welding or threaded engagement. Outer surface of distal housing portion 56 includes a collar 58 at its proximal open end. Housing 52 further includes one or more grooves (not shown) for receiving one or more outer resilient sealing members 60. As seen in Fig. 8, sealing members 60 are arranged in parallel and are spaced from one another by the outer wall of housing 52. As with connector 22, connector 24 includes an attachment member 62 that proximally extends from the proximal housing portion 54. Attachment member 62 defines an interior flow path that communicates with the interior of housing 52. The outer surface of attachment member 62 may be likewise contoured or otherwise sized and shaped to receive the lumen of tubing 16 associated with processing set 12 and to secure the tubing end to connector 24. Thus, as shown in Figs. 7 and 8, attachment member 62 may include a barb for press fitting tube 16 onto attachment member 62. In lieu of a barb, as shown, attachment member 54 may include ribs, fins, luer lock or threads for securing tubing 16.

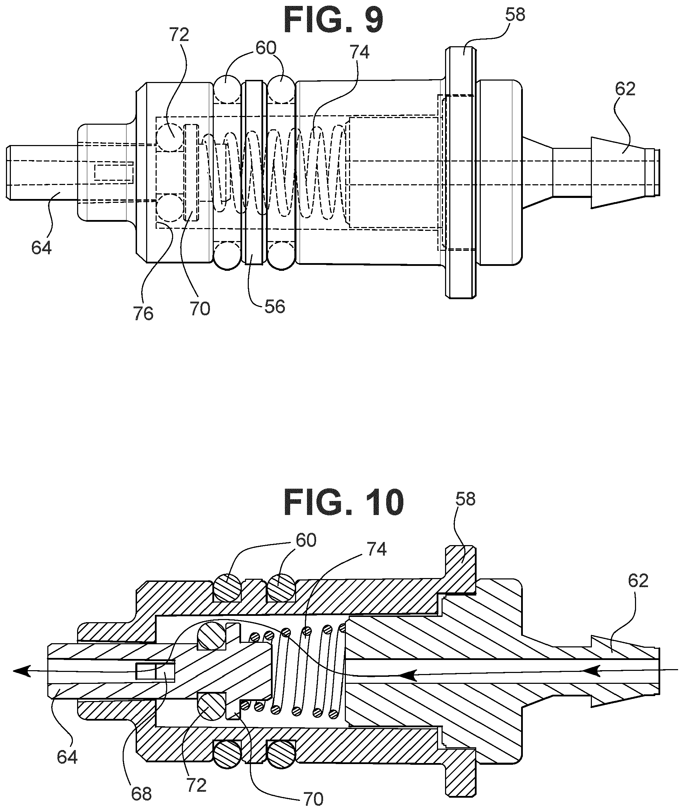

Fig. 8 and 9 show the internal components of connector 24. Like connector 22, connector 24 includes a plunger 64 with an internal flow path 66 and an aperture 68 in the wall of plunger 64. Plunger 64 further includes a flange or collar 70 that supports sealing member 72. In one embodiment, sealing member 72 is a resilient O-ring. Plunger 64 is attached to spring 74. As seen in Fig. 9, when spring 74 is in its expanded condition, sealing member 72 is pressed against wall 76 of the passageway through which plunger 64 extends and creates a liquid tight seal, thereby preventing liquid flow through connector 24. When spring 74 is compressed, plunger 64 moves in a proximal direction thereby opening a flow path (by moving the sealing member 72) through second connector 24 and allowing flow through aperture 68, as shown in Fig. 10.

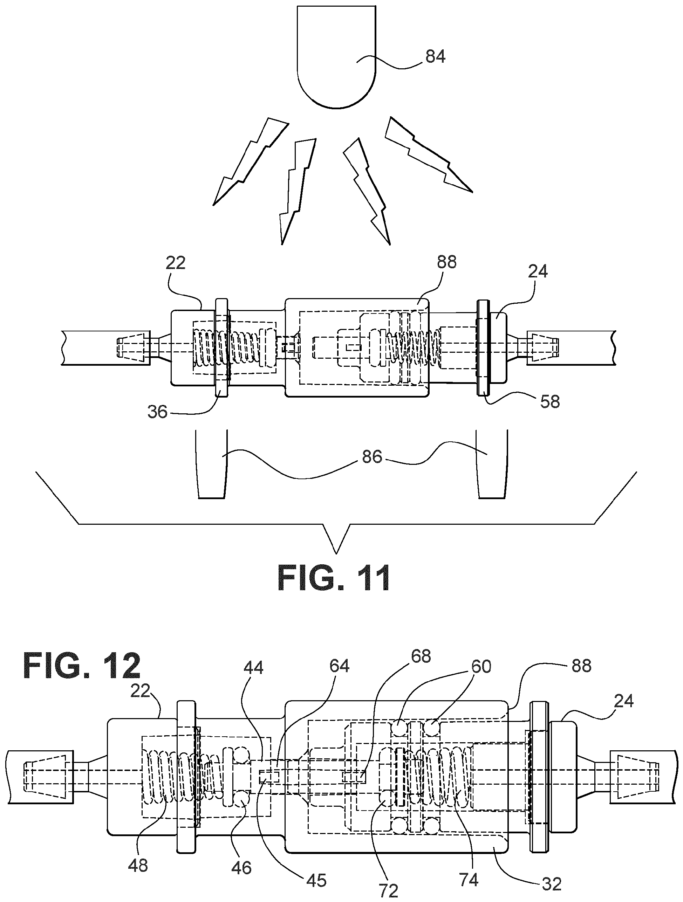

Figs. 11-14 show the steps in the method of establishing a reversible sterile flow path between two processing sets, sterilizing the flow path, and undoing the flow path in accordance with the system and devices disclosed herein. Inasmuch as the sterilizing step will involve the exposure of the established flow path to sterilizing light from a light source, it will be understood that the system will include, and the method will be carried out in an enclosed light chamber 82 as generally shown in Fig. 11. Light chamber 82 will typically include a light source 84 and a movable holding assembly 86 in which connectors 22 and 24 can be mounted and selectively moved toward and away from each other by an actuation of a motor, cam assembly, lead screws or other driving means (not shown). Thus, light chamber 82 may be part of a stationary, table-top or portable irradiation device (light box) as shown, for example, in U.S. provisional patent application identified by Attorney Docket Number F-6938 (9360-0735), filed simultaneously herewith and incorporated by reference herein. In a further embodiment, the irradiation chamber may be incorporated in or integrated with a blood or biological fluid processing device or system.

Thus, turning now to Fig. 11 a system for establishing a sterile fluid connection between a first fluid processing set and a second fluid processing set is shown. The system may include a light chamber 82 housed in a stationary or portable device that also includes light source 84 and a holding assembly 86 for receiving connectors 22 and 24. In one embodiment, holding assembly 86 may include a pair of holders configured to receive connectors 22 and 24. In a more particular embodiment, holders of holding assembly may include slots for receiving collars 36 and 58 of connectors 22 and 24. Connectors 22 and 24 to which the open ends of tubing 16 and 20 are attached may be mounted and fixed within the slots of the holders 86. As further shown in Fig. 11, the distal ends of connectors 22 and 24, respectively are mounted in a face-to-face to arrangement.

As connectors 22 and 24 are advanced toward each other, distal end of connector 24 enters receiver 32 of connector 22. Resilient sealing members 60 of second connector 24 may have a slightly larger diameter than the inside diameter of receiver chamber 34 thereby providing an air-tight seal between first and second connectors 22 and 24. Once resilient sealing members 60 of connector have entered receiver 32 and cleared the outermost distal rim 88 of receiver 32, connection site and the flow path established thereby are ready to be sterilized.

Connectors 22 and 24 and, more particularly, housings 25 and 52, respectively are each preferably (a) made of a material and (b) have a housing wall thickness that can be penetrated by a sterilizing medium to effectively sterilize the interior of the of the housing and the connection site established by the bringing together of connectors 22 and 24. Where the sterilizing medium is light, connector housings 25 and 52 should be made of a material that is transparent to the sterilizing wavelength of the light to be delivered. In accordance with the systems and device disclosed herein, the light source may be ultraviolet C (UV-C) light and the connector housings may be made of a polymeric material such as, but not limited to cyclic olefin copolymers. A suitable housing wall thickness for connectors may be between 0.5 mm and 5mm. Where light of a different wavelength is used, or a different sterilizing medium altogether is used, housings made of different materials and having different housing thicknesses may be used.

In the embodiment where the sterilizing medium is UV-C and the connector housings are made of cyclic olefin copolymer, the duration of irradiation by the light source may be anywhere between 0.1sec and 300 sec. It will be understood that the duration of irradiation may depend, at least in part, on the light intensity. Of course, the duration of irradiation may be affected by the size and contour of the light chamber, light intensity, and the positioning of the connection site relative to the incident light as well as other factors. While tubing ends of processing sets 12 and 11 that are attached at attachment members 30 and 54 may be exposed to the sterilizing radiation, in general the sets 12 and 11, including the tubing ends are typically pre-sterilized.

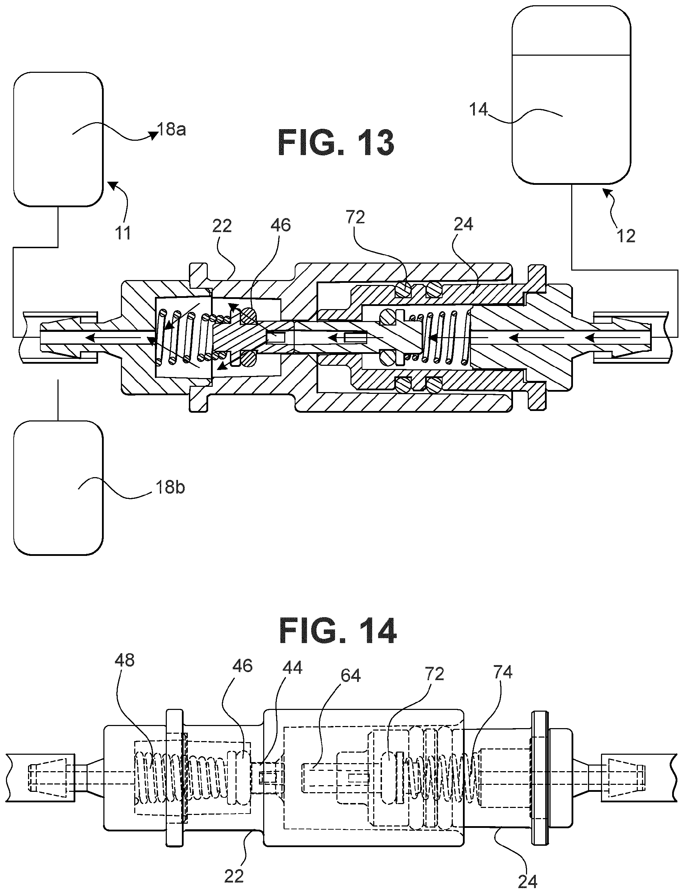

Once sterilization of the connection site is complete, connectors are further advanced toward each other as shown in Fig. 12. As connectors 22 and 24 are further moved toward each other, the respective distal ends of plungers 44 and 64 contact each other and as they do, engage springs 48 and 74 and move internal sealing members 46 and 72 from their sealing position to open-flow positions. Apertures 45 and 68 in plungers 44 and 64 are placed in open fluid communication with the interiors within the respective connectors 22 and 24 thereby establishing an open flow path between processing sets 12 and 11 as shown in Fig. 13. Fluid may now be transferred from processing set 12 to processing set 11 through a sterile flow path.

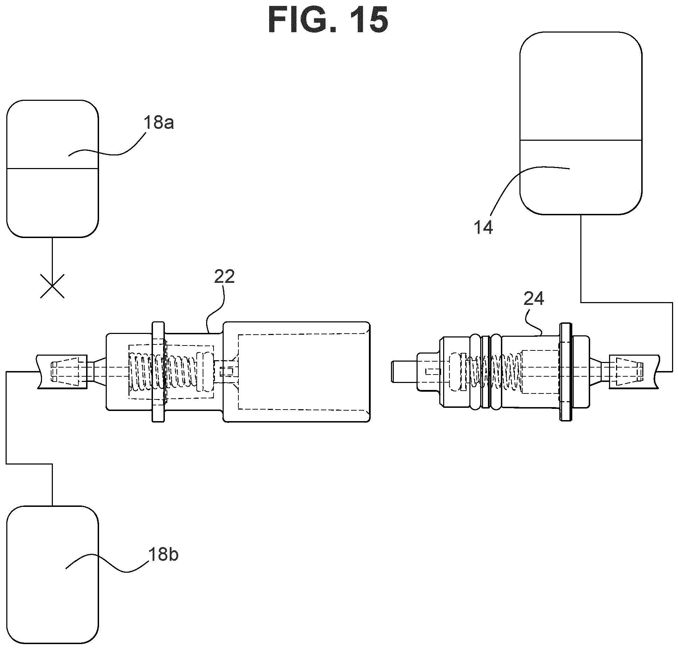

After fluid transfer, connectors 22 and 24 may be disengaged as shown in Fig. 14. As connectors are retracted, springs 48 and 74 return internal sealing members 46 and 72 to their sealed positions, with sealing members pressed against the passageway between connectors 22 and 24 and apertures 45 and 68 blocked from open flow communication. As connectors are further retracted and fully released from contact, they are ready for the next sterile fluid transfer as shown in Fig. 15. For example, if fluid from source container 14 is to be dispensed to additional receptacles 18(b) (and 18(c) etc.), wherein each additional receptacle likewise includes a pre-attached and pre-sterilized connector of the type described herein. Connectors 22 and 24 may be brought together in the manner described above and the engagement and sterilization may be repeated.

The advantages of the disclosed connection system as compared to existing practices is the ability to perform multiple repeatable sterile fluid flow connections between two fluids using a single connection system. In this manner one fluid pathway can be replaced by another and the same connection system can be repeatedly used to create additional sterile fluid pathways. This method does not require manual user disinfection by swabs or the use of heater wafers and each connector can be reused and sterilized as the user sees fit.

The application of repeatable sterile connections using these systems, devices, and methods described herein are especially useful in distributing high volume collected biological components into multiple bags or fluid pathways without permanently bonding each fluid pathways. The reversible sterile connection system reduces hands-on time by the technician and eliminates any potential product loss due to standard heated wafer sterile connection techniques. As described above, the connection system of this disclosure may be used with or within laboratory devices to distribute biological components into individual units for medical use.

### Other Examples

Aspects of the present subject matter described above may be beneficial alone or in combination with one or more other Aspects, as described below.

Aspect 1. A system for establishing a sterile fluid connection between a first fluid processing set and a second fluid processing set. The system includes a first connector configured for attachment with a first fluid processing set and a second connector configured for attachment with a second fluid processing set. The first connector includes a housing having a receiver at a distal end of the first connector, the receiver includes an open end and defining a chamber configured to receive at least a portion of the second connector in liquid-tight engagement. A light source is in proximity to the first and second connectors.

Aspect 2. The system of Aspect 1 wherein the first connector includes an openable flow path between the receiver and the first fluid processing set.

Aspect 3. The system of any one of Aspects 1 and 2 wherein the second connector includes an openable flow path with the second fluid processing set.

Aspect 4. The system of any one of Aspects 1 through 3 wherein the first connector includes an attachment member located at a proximal end of the first connector and is configured to receive a tube of the first fluid processing set.

Aspect 5. The system of any one of Aspects 1 through 4 wherein the second connector includes an attachment member configured to receive a tube of the second fluid processing set.

Aspect 6. The system of any one of Aspects 1 through 5 wherein the first connector includes a sealing member movable along a central axis.

Aspect 7. The system of Aspect 6 wherein the first connector includes a plunger, the plunger of the first connector includes a distal end and an opening in the distal end.

Aspect 8. The system of any one of Aspects 1 through 7 wherein the second connector includes a housing having a plunger and sealing member movable within the housing.

Aspect 9. The system of Aspect 8 wherein the plunger of the second connector includes a distal end and an opening in the distal end.

Aspect 10. The system of any one of Aspects 8 through 9 wherein the first connector housing and the second connector housing are made of a transparent material.

Aspect 11. The system of Aspect 10 wherein the light source emits light of a wavelength selected to effectively sterilize the first and second connectors when said connectors are in liquid-tight engagement.

Aspect 12. The system of any one of Aspects 1 through 11 wherein the light source emits ultraviolet C (UV-C) light.

Aspect 13. The system of any one of Aspects 10 through 12 further comprising an enclosed irradiation chamber configured to receive the first and second connectors and an actuator for activating the light source.

Aspect 14. The system of any one of Aspect 13 further comprising a movable holding assembly for aligning the first and second connectors in a facing arrangement.

Aspect 15. A connector for establishing a reversible sterile fluid connection between a first tube and a second tube. The connector includes a body having a proximal end portion and a distal end portion. The body has a central axis and a plunger moveable within the body. A sealing member is associated and moveable with the plunger from a first sealing position to a second open flow position. A tube attachment member is located at the proximal end portion of the body and an openable flow path is located between the attachment member and the plunger.

Aspect 16. The connector of Aspect 15 wherein the body is generally cylindrical and is made of a material that is transparent to light of a selected wavelength.

Aspect 17. The connector of any one of Claims 15 and 16 wherein the plunger includes an open aperture.

Aspect 18. The connector of Aspect 17 including a spring for actuating movement of the plunger and the sealing member.

Aspect 19. The connector of Aspect 17 wherein the body includes a collar proximally spaced from the sealing member.

Aspect 20. The connector of any one of Aspects 15 and 16 wherein the connector includes at least one or more additional sealing members. The at least one or more additional sealing members are sized and shaped to provide fluid-tight engagement with another connector.

Aspect 21. The connector of any one of Aspects 15 and 16 wherein the distal end portion defines a receiver including an inner surface defining a receiving chamber configured to receive at least a portion of another connector, the receiver further including a distal open end.

Aspect 22. A method of establishing a sterile fluid connection between two or more fluid processing sets, wherein each of sets includes a tube defining a flow path. The method includes attaching a tube of a first fluid processing set to a proximal end of a first connector that includes a plunger with a distal eyelet and a sealing member. The sealing member is associated and moveable with the plunger from a first sealing position to a second open flow position. The method further includes attaching a tube of a second processing set to a proximal end of a second connector. The second connector includes a plunger with a distal eyelet and a sealing member, the sealing member being associated and moveable with the plunger from a first sealing position to a second open flow position. The method includes aligning a distal end of the first connector plunger with a distal end of the second connector plunger in a face-to-face relationship; contacting said distal ends of said first and second connector plungers; moving each of the sealing members of the first and second connectors from the first sealing position to said open flow position; exposing the first and second connectors to light from a light source for a selected period of time. The light is sufficient to sterilize interiors of said first and second connectors; and flowing fluid from a first processing set through said first and second connectors to the second processing set.

Aspect 23. The method of Aspect 22 further including returning each of the sealing members from said open flow position back to the sealing positions.

Aspect 24. The method of any one of Aspects 22 and 23 including introducing a portion of first connector into a receiver of a second connector.

Aspect 25. The method of Aspect 24 including forming an air-tight seal between the portion of said first connector and an inner surface of the receiver.

Aspect 26. The method of any one of Aspects 22 through 25 further including detaching at least one of the connectors from its processing set and attaching the detached connector to a different processing set.

Aspect 27. The method of Aspect 26 further including repeating the steps of Aspect 22.

## Claims

1. A system for establishing a sterile fluid connection between a first fluid processing set and a second fluid processing set comprising:
a first connector configured for attachment with a first fluid processing set;
a second connector configured for attachment with a second processing set;
said first connector including a housing having a receiver at a distal end of said first connector, said receiver including an open end and defining a chamber configured to receive at least a portion of said second connector in liquid-tight engagement;
a light source in proximity said first and second connectors.

2. The system of Claim 1 wherein said first connector includes an openable flow path between said receiver and said first fluid processing set and said second connector includes an openable flow path with said second fluid processing set.

3. The system of any one of Claims 1 through 2 wherein said first connector includes an attachment member located at a proximal end of said first connector and configured to receive a tube of said first fluid processing set and said second connector includes an attachment member configured to receive a tube of said second fluid processing set.

4. The system of any one of Claims 1 through 3 wherein said second connector includes a housing having a plunger and sealing member movable within said housing, said plunger of said second connector includes a distal end and an opening in the distal end .

5. The system of any one of Claims 1 through 4 wherein said first connector housing and said second connector housing are made of a transparent material and wherein said light source emits light of a wavelength selected to effectively sterilize said first and second connectors when said connectors are in liquid-tight engagement.

6. The system of any one of Claims 1 through 5 further comprising an enclosed irradiation chamber configured to receive said first and second connectors and an actuator for activating said light source, said system further comprising a movable holding assembly for aligning said first and second connectors in a facing arrangement.

7. A connector for establishing a reversible sterile fluid connection between a first tube and a second tube, said connector comprising:
a. a body having a proximal end portion and a distal end portion, said body having a central axis;
b. a plunger moveable within said body;
c. a sealing member associated and moveable with said plunger from a first sealing position to a second open flow position;
d. a tube attachment member located at said proximal end portion of said body; and
e. an openable flow path between said attachment member and said plunger.

8. The connector of Claim 7 wherein said body is generally cylindrical and is made of a material that is transparent to light of a selected wavelength.

9. The connector of any one of Claims 7 and 8 wherein said plunger includes an open aperture and a spring for actuating movement of said plunger and said sealing member.

10. The connector of any one of Claims 7 and 9 wherein said connector comprises at least one or more additional sealing members, said at least one or more additional sealing members sized and shaped to provide fluid-tight engagement with another connector.

11. The connector of any one of Claims 7 through 9 wherein said distal end portion defines a receiver including an inner surface defining a receiving chamber configured to receive at least a portion of another connector, said receiver further comprising a distal open end.

12. A method of establishing a sterile fluid connection between two or more fluid processing sets, each of said sets including a tube defining a flow path, said method comprising:
a. attaching a tube of a first fluid processing set to a proximal end of a first connector, said first connector including a plunger with a distal eyelet and a sealing member, the sealing member associated and moveable with said plunger from a first sealing position to a second open flow position;
b. attaching a tube of a second processing set to a proximal end of a second connector said first connector including a plunger with a distal eyelet and a sealing member, the sealing member associated and moveable with said plunger from a first sealing position to a second open flow position;
c. aligning a distal end of the first connector plunger with a distal end of the second connector plunger in face-to-face relationship;
d. contacting said distal ends of said first and second connector plungers;
e. moving each of said sealing members of said first and second connectors from said first sealing position to said open flow position;
f. exposing said first and second connectors to light from a light source for a selected period of time, said light being sufficient to sterilize interiors of said first and second connectors; and
g. flowing fluid from a first processing set through said first and second connectors to said second processing set.

13. The method of Claim 12 further comprising returning each of said sealing members from said open flow position back to said sealing positions.

14. The method of any one of Claims 12 and 13 comprising introducing a portion of first connector into a receiver of a second connector.

15. The method of any one of Claims 12 through 14 further comprising detaching at least one of said connectors from its processing set and attaching said detached connector to a different processing set.
